# EUROPEAN PATENT APPLICATION

(11) **EP 4 652 931 A1**
(43) Date of publication of application: **26.11.2025**
(21) Application number: 24178024.6
(22) Date of filing: 24.05.2024
(51) Int. Cl.: A61B 5/18, A61B 5/00, G06N 3/0464, G06V 20/59

(54) **COMPUTER-IMPLEMENTED METHOD BASED ON A CONVOLUTIONAL NEURAL NETWORK TO DETECT A DROWSINESS STATE OF A DRIVER**

(71) Applicant: Valeo Comfort and Driving Assistance, 94000 Créteil (FR)
(72) Inventor: CHELLALI, Mohamed-Zaki, 94000 Créteil (FR); JAVANMARDI, Setareh, 94000 Créteil (FR); CHRETIEN, Clement, 94000 Créteil (FR); JAVID, Gelareh, 94000 Créteil (FR); CARON, Sevil, 94000 Créteil (FR)
(74) Representative: Delplanque, Arnaud

(57) **Abstract**

The invention relates to a first computer-implemented method (1) to detect a state (st) of a driver of a vehicle among a drowsiness state (st1) and a non-drowsiness state (st2), said first computer-implemented method (1) comprising :
- receiving as an input by a convolutional neural network (CNN) a first input dataset (D1) comprising first data (d1), said convolutional neural network (CNN) comprising a set of hyperparameters (Hp),
- processing said first data (d1) through a plurality of layers (L) of said convolutional neural network (CNN),
- generating by said convolutional neural network (CNN) first outputs (01) that are a classification of the driver's state (st) among the drowsiness state (st1) and the non-drowsiness state (st2).

## Description

### TECHNICAL FIELD

The present invention relates to a first computer-implemented method to detect a state of a driver of a vehicle among a drowsiness state and a non-drowsiness state. Such a first computer-implemented method may be used, but not exclusively, in the automotive domain.

### BACKGROUND OF THE INVENTION

In the automotive domain, a first computer-implemented method to detect a state of a driver of a vehicle among a drowsiness state and a non-drowsiness state, well-known by the person skilled in the art, comprises:
- receiving by an electronic control unit of the vehicle a plurality of first data,
- upon some rules and said first data, deducing by the electronic control unit if the driver in a drowsiness state or in a non-drowsiness state.

Such first computer-implement method is implemented in a driver monitoring system also referred to as DMS. The first data of the input dataset are :
- vehicle signals such as the speed of the vehicle, or acceleration of the vehicle, and
- facial features of the driver such as the eyes and eyelids, and/or
- biological signals of the driver such as the heart rate.

Some vehicle signals such as lane markings are acquired by a frontal exterior camera. The facial features are acquired by a camera inside the vehicle and the biological signals of the driver are acquired by a sensor that provides an electrocardiogram signal of the driver.

Based on a combination of these first data, the first computer-implemented method detects if the driver is drowsy or not.

One problem of this prior art is that the detection is based on specific rules on different combinations of these first data which can be rather complex and costly due to the use of the camera and the sensor.

### SUMMARY OF THE INVENTION

It is an object of the invention to provide a first computer-implemented method to detect a state of a driver of a vehicle among a drowsiness state and a non-drowsiness state, which resolves the problem above-stated.

To this end, it is provided a first computer-implemented method to detect a state of a driver of a vehicle among a drowsiness state and a non-drowsiness state, said first computer-implemented method comprising :
- receiving as an input by a convolutional neural network a first input dataset comprising first data, said convolutional neural network comprising a set of hyperparameters,
- processing said first data through a plurality of layers of said convolutional neural network,
- generating by said convolutional neural network first outputs that are a classification of the driver's state among the drowsiness state and the non-drowsiness state.

As we will see in details later in the description, thanks to the use of the convolutional neural network to detect the state of the driver, one doesn't need to implement complex combinations of rules to determine the state of a driver. Moreover, one doesn't need any more facial features or biological signals which reduces the complexity of the detection of the state of a driver, and thus one can suppress the camera in the vehicle's interior and the sensor for the biological signals so as to reduce the cost.

According to non-limitative embodiments of the invention, the first computer-implemented method in accordance with the invention further comprises the following characteristics.

In a non-limitative embodiment, said first data are :
- a vehicle speed, and/or
- a vehicle steering wheel angle, and/or
- a vehicle brake pedal pressure, and/or
- a vehicle acceleration pedal pressure, and/or
- a vehicle yaw rate, and/or
- a vehicle position on a road lane.

In a non-limitative embodiment, said first input dataset comprises about a number of sequences of 300 by 6 elements, each sequence being acquired during a history of one 1 minute.

In a non-limitative embodiment, the first input dataset is updated each N milliseconds using a sliding window, with N integer.

In a non-limitative embodiment, N is subsequently equal to 200 milliseconds.

In a non-limitative embodiment, said first data are non-annotated data.

In a non-limitative embodiment, said hyperparameters are :
- a loss function that is a binary focal cross entropy,
- an optimizer that is an Adam optimizer,
- a learning rate of 10e-4
- an epoch number of 23.

In a non-limitative embodiment, said first computer-implemented method further comprises normalizing the first data using MinMax normalization.

In a non-limitative embodiment, said convolutional neural network comprises:
- one input convolutional layer,
- three convolutional layers,
- one fully connected layer,
- one final connected output layer.

In a non-limitative embodiment, said three convolutional layers use each a Relu activation function and by a batch normalization layer, each three convolutional layers comprising respectively 12, 10 and 11 filters of size 3, - said fully connected layer Full_L of 15 units with a Relu activation function, said final connected output layer has 2 units and is using a Softmax function.

In a non-limitative embodiment, said convolutional neural network further comprises :
- one dropout layer, and
- one max pooling layer.

In a non-limitative embodiment, said convolutional neural network further comprises an additional fully connected layer that is a flatten layer that is applied before the fully connected layer.

In a non-limitative embodiment, a backpropagation that is applied on the whole layers so as to propagate an error gradient from the final connected output layer to the input convolutional layer.

In a non-limitative embodiment, said dropout layer has a drop rate of 0.5, and said max pooling layer has a pool size of 2.

It is also provided a second computer-implemented method for warning a driver of a vehicle of his drowsiness state, said second computer-implemented method comprising :
- receiving as an input for a convolutional neural network a first input dataset comprising first data, said convolutional neural network comprising a set of hyperparameters,
- running an inference of said convolutional neural network so as to execute said first computer-implemented method according to any of the preceding characteristics so that said inference convolutional neural network generates first outputs that are a classification of the driver's state among the drowsiness state and the non-drowsiness state,
- if the driver's state is classified as the drowsy state, sending a first warning information to a human machine interface of the vehicle.

According to non-limitative embodiments of the invention, the second computer-implemented method in accordance with the invention further comprises the following characteristics.

In a non-limitative embodiment, said second computer-implemented method further comprising :
- each time the inference convolutional neural network generates a classification that corresponds to a drowsiness state, a filtering counter is increased by the electronic control unit with a filtering bonus,
- each time the inference convolutional neural network generates a classification that corresponds to a non-drowsiness state, the filtering counter is decreased by the electronic control unit with a filtering malus,
- if the filtering counter is above a first filtering threshold, classifying the driver's state as the drowsy state.

It is also provided an electronic control unit for a vehicle, wherein said electronic control unit being configured to :
- receive as an input for a convolutional neural network a first input dataset comprising first data, said convolutional neural network comprising a set of hyperparameters,
- run an inference of said convolutional neural network so that said convolutional neural network generates first outputs that are a classification of the driver's state among the drowsiness state and the non-drowsiness state,
- if the driver's state is classified as the drowsiness state, send a first warning information to a human machine interface of the vehicle.

It is also provided a computer program product for a computer, comprising a set of instructions, which when loaded into said computer, causes the computer to carry out the second computer-implemented method according to any of the preceding characteristics.

It is also provided a non-transitory machine-readable medium having instructions stored therein, which when executed by a processor, cause the processor to perform the second computer-implemented method according to any of the of the preceding characteristics.

### BRIEF DESCRIPTION OF THE FIGURES

Some embodiments of methods and/or apparatus in accordance with embodiments of the present invention are now described, by way of example only, and with reference to the accompanying drawings, in which:
Figure 1 is a schematic diagram that illustrates a first computer-implemented method to detect a state of a driver of a vehicle among a drowsiness state and a non-drowsiness state, said first computer-implemented method using a convolutional neural network, according to a non-limitative embodiment of the invention,
Figure 2 is a schematic diagram that illustrates the first computer-implemented method of figure 1, said first computer-implemented method comprising further steps according to non-limitative embodiments,
Figure 3 illustrates a non-limitative embodiment of a first input dataset received by said convolutional neural network during a step of said first computer-implemented method of figures 1 and 2,
Figure 4a is a diagram of a first part of the convolutional neural network used within the first computer-implemented method of figures 1 and 2, according to a non-limitative embodiment,
Figure 4b is a diagram of a second part of the convolutional neural network used within the first computer-implemented method of figures 1 and 2, according to a non-limitative embodiment,
Figure 5 is a schematic diagram that illustrates a first non-limitative embodiment of a second computer-implemented method for warning a driver of a vehicle of his drowsiness state,
Figure 6 is a schematic diagram that illustrates a second non-limitative embodiment of a second computer-implemented method for warning a driver of a vehicle of his drowsiness state,
Figure 7 is a schematic diagram that illustrates a non-limitative embodiment of an electronic control unit of a vehicle that is configured to run the convolutional neural network of figures 4a and 4b.
Figure 8 is a schematic diagram that illustrates a buffer of the electronic control unit of figure 7.

### DESCRIPTION OF EMBODIMENTS OF THE INVENTION

In the following description, well-known functions or constructions by the man skilled in the art are not described in detail since they would obscure the invention in unnecessary detail.

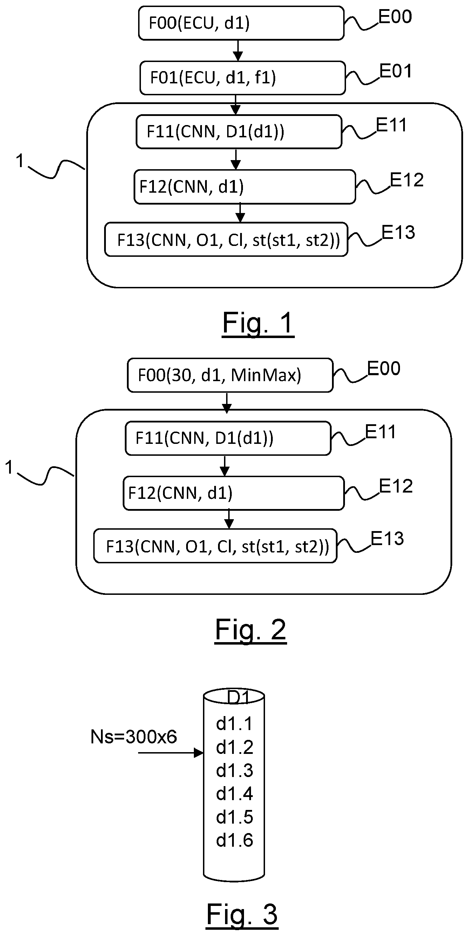

The present invention relates to a first computer-implemented method 1 to detect a state st of a driver of a vehicle 3 among a drowsiness state st1 and a non-drowsiness state st2, said first computer-implemented method 1 being described in reference to figures 1 and 2, according to non-limitative embodiments.

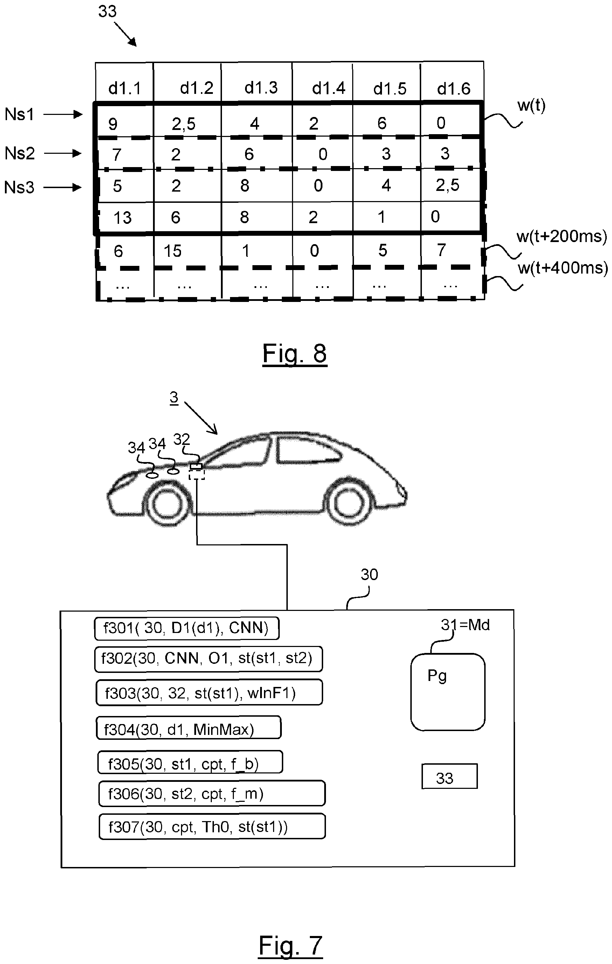

In non-limitative embodiments, the vehicle 3 illustrated in figure 7 is a motor vehicle, an electrical vehicle or a hybrid vehicle. The vehicle 3 comprises an electronic control unit 30, a human machine interface 32 and sensors 34 illustrated in figure 7.

The first computer-implemented method 1 is based on a convolutional neural network also referred to as CNN model or simply CNN in the following. The convolutional neural network is run on the electronic control unit 30. When it is run, the CNN is called an inference CNN model or simply inference model or inference CNN. In a non-limitative embodiment, the CNN is a 1D-CNN. It means that it receives as an input a vector instead of a matrix for a multi-dimensional CNN.

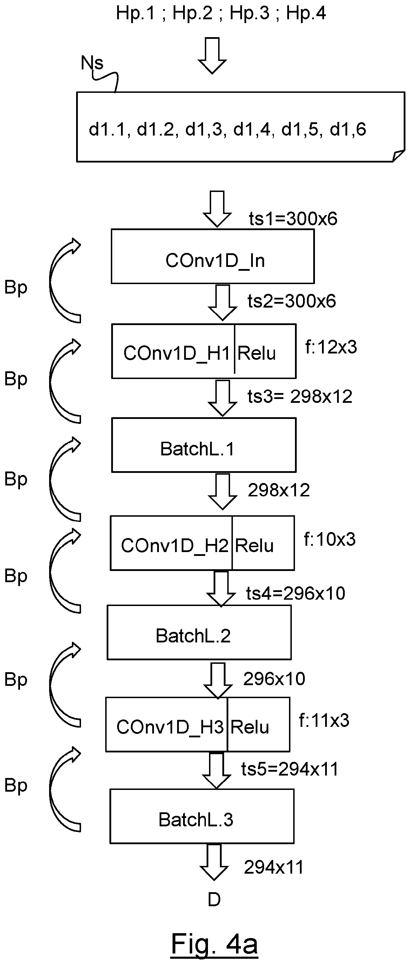

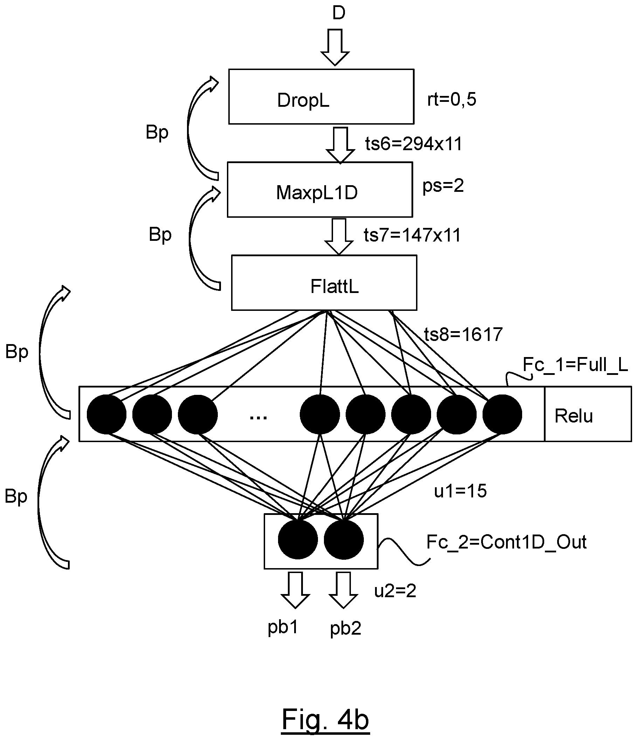

As illustrated in figures 4a and 4b, the CNN is composed of a plurality of layers among which:
- one input convolutional layer Conv1D_In as illustrated in figure 4a,
- three convolutional layers Conv1D_H1, Conv1D_H2, Conv1D_H3 as illustrated in figure 4a,
- one fully connected layer Full_L as illustrated in figure 4b,
- one final connected output layer Cont1D_Out as illustrated in figure 4b.

It is to be noted that the fully connected layer Full_L and the final connected output layer Cont1D_Out are dense layers. It is to be noted that all layers between the input convolutional layer Conv1D_In layer and the final connected output layer Cont1D_Out layer are considered as hidden layers.

As illustrated in figures 4a and 4b, the CNN comprises a plurality of filters f related to different layers. In a non-limitative embodiment, the size of the filters f is 3. A filter is also called kernel. It is used to extract information from a previous layer and create a feature map that is used in the next layer.

A filter f is small matrix of weights that slides over the input tensor of a layer. It slides from top to bottom. It performs element wise-multiplication with the part of the input it is currently on, and sums up all the results into a single output data.

The different layers are described in details hereinafter.

The input convolutional layer Conv1D_In is configured to receive a first input tensor ts1 of size 300x6 and to output a first output tensor ts2 of size 300x6 which is also called second input tensor ts2 as it serves as an input tensor for the first convolutional layer Conv1D_H1. Hence, the first input tensor ts1 comprises 300 vectors of 6 elements each, one element being a sample of a first data d1 that will be described in the following.

The three convolutional layers Conv1D_H1, Conv1D_H2, Conv1D_H3 are 1D convolutional layers. They are followed each by respectively a batch normalization layer BatchL.1, BatchL.2, BatchL.3. Each three convolutional layers comprising respectively 12, 10 and 11 filters f of size 3. The three convolutional layers Conv1D_H1, Conv1D_H2, Conv1D_H3 are used to extract various features (also called patterns) from the first input dataset D1 and to obtain different features map as output tensors which are respectively referred to as second output tensor ts3, third output tensor ts4 and fourth output tensor ts5 in figure 4a. For simplicity, they will be called output tensor ts3, output tensor ts4 and output tensor ts5 in the following. The output tensors ts3, ts4 and ts5 are also called convolved feature maps.

The three convolutional layers Conv1D_H1, Conv1D_H2, Conv1D_H3 use an activation function. In a non-limitative embodiment, the activation function is a Relu activation function. It permits introducing non-linearity to the CNN. It allows the CNN to learn complex, non-linear relationships in the input tensors.

The fully connected layer Full_L has 15 units. It means it has a set of 15 neurons. The fully connected layer Full_L is made up of a set of neurons, where each neuron is fully connected to all neurons in the previous layer. It uses a Relu activation function.

The final connected output layer Cont1D_Out has 2 units and is using a Softmax function. It means it has a set of 2 neurons.

In a non-limitative embodiment, as illustrated in figure 4b, the CNN further comprises :
- one dropout layer DropL, and
- one max pooling layer MaxpL1D.

The dropout layer DropL follows the third convolutional layers Conv1D_H3. In a non-limitative embodiment, the dropout layer DropL has a drop rate rt of 0.5.

The max pooling layer MaxpL1D follows the dropout layer DropL. In a non-limitative embodiment, the max pooling layer MaxpL1D has a pool size ps of 2.

In a non-limitative embodiment, as illustrated in figure 4b, the CNN further comprises a supplementary fully connected layer that is a flatten layer FlattL that is applied before the fully connected layer Full_L and that follows the max pooling layer MaxpL1D.

The first computer-implemented method 1 comprises the following steps described in figure 1 according to a non-limitative embodiment.

As we will see, the CNN uses first input data d1 also called first data d1, to be processed through its different layers. The first input data d1 are part of a first input dataset D1 also called first dataset D1 or dataset D1. The dataset D1 is illustrated in figure 3. In a non-limitative embodiment, the first input dataset D1 comprises a number of sequences Ns of 300 by 6 elements.

In a non-limitative embodiment, Ns = 166939. In a non-limitative embodiment, the CNN has all the layers mentioned above including the dropout layer DropL, the max pooling layer MaxpL1D and the flatten layer FlattL.

In a first step E11 illustrated F11(CNN, D1(d1)) in figure 1, the CNN receives as input the first input dataset D1 of first data d1.

The first data d1 are raw data. They are non-annotated data. When running the inference CNN, there is no annotated data. In a non-limitative embodiment, the first data d1 are signals transmitted by a Controller Area Network of the vehicle when the vehicle 3 is moving, also called CAN or CAN bus, to the electronic control unit 30. The signals are acquired by the sensors 34 of the vehicle 3. In the non-limitative example illustrated in figure 7, there are two sensors 34 illustrated.

In a non-limitative embodiment, as illustrated in figure 3, the first data d1 are of the type :
- a vehicle speed d1.1, and/or
- a vehicle steering wheel angle d1.2, and/or
- a vehicle brake pedal pressure d1.3, and/or
- a vehicle acceleration pedal pressure d1.4, and/or
- a vehicle yaw rate d1.5, and/or
- a vehicle position on a road lane d1.6.

In a non-limitative example, there are six first data d1 of each type as illustrated in figure 3.

In a non-limitative embodiment, each 200ms, the electronic control unit 30 of the vehicle 3 sends 6 raw data that represent the different signals of the sensors 34 as d1.1, d1.2, d1.3 ... d1.6. And the CNN takes a set of 300 elements which represents 1 minute, so that the input tensor ts1 is of [300 x 6]. In other words, a sequence is acquired during a history of one 1 minute.

In a non-limitative embodiment, the first input dataset D1 is updated each N milliseconds using a sliding window w, with N integer. In a non-limitative example, when each CAN signal arrived each 200 milliseconds, N is subsequently equal to 200 milliseconds. As the number of sequences Ns is of 300 by 6 elements, hence, each element of the 300 is acquired in 200 ms, so 300 elements represents 1 minute. This value of 200 ms is a tradeoff choice to keep information precision with less calculations. It means that each 200 ms, there is a new sample of each first data d1 that is received It permits to have a history of the state of the driver which is up to date. In practical, to do so, one uses a buffer 33 (illustrated in figures 7 and 8) of the electronic control unit 30. In this case, the size of the buffer is 300. The sliding window w illustrated in figure 8 means that at an instant t the buffer 33 has 300 elements from 1 to 300 which is a first sequence Ns1, then at instant t+200ms 300 elements from 2 to 301 which is a second sequence Ns2, then at instant t+400ms 300 elements from 3 to 302 which is a third sequence Ns3 etc. The sliding window w permits to discard the oldest value, to shift and to add a new sample of a first data d1 in the buffer 33.

In this non-limitative example, when the first input dataset D1 comprises a number of sequences Ns of 300 by 6 elements, it means that the 300 elements received correspond to 1 minute. If six different types of first data d1 are considered (from d1.1 to d1.6), it means that one takes the history during 1 minute of these 6 first data d1. The 300 elements represent the very last minute. The 300 elements acquired during 1 minute represent a local pattern of the state of the driver.

In step E12 illustrated F12(CNN, d1) in figure 1, the first data d1 are processed through the plurality of layers of the CNN.

It is to be noted that all the layers of the CNN and the final connected output layer Cont1D_Out have a 2D output tensor.

The first layers capture simple patterns and the deeper we goes the more complex patterns we lean. For example convolutional layer Conv1D-H2 learns more complex patterns than convolutional layer Conv1D-H1, and convolutional layer Conv1D-H3 learns more core complex than convolutional layer Conv1D-H2 and so on.

It is to be noted that the CNN comprises a set of hyperparameters Hp. As illustrated in figure 4a, in a non-limitative embodiment, the parameters Hp are :
- a loss function Hp.1 that is a binary focal cross entropy,
- an optimizer Hp.2 that is an Adam optimizer,
- a learning rate Hp.3 of 10e-4,
- an epoch number Hp.4 of 23.

These hyperparameters Hp have been set so that the objective of detecting the state st of the driver with good performances is attained.

The first input tensor ts1 of the input convolutional layer Conv1D_In is of 300x6. It is a sequence Ns of first data d1. In the non-limitative given example, each 1 minute, this sequence Ns changes and the input tensor ts1 changes.

The first output tensor ts2 is received as an input tensor by the first hidden layer which is the first convolutional layer Conv1D_H1 of the three convolutional layers Conv1D_H1, Conv1D_H2, Conv1D_H3 mentioned before.

Batch-normalization is done between different layers, and permits to have higher learning rates, making learning easier. It is done along mini-batches instead of the full first dataset D1.

The batch normalization layer BatchL.1 that follows permits to normalizes the feature map (aka the result of the COnv1D_H1) that is a tensor of 298x12 and that goes through this first convolutional layer Conv1D_H1.

The output tensor ts3 is of size 298x12 and serves as an input tensor for the second hidden layer which is the second convolutional layer Conv1D_H2. As, we can see, from the first convolutional layer Conv1D_H1 to the second convolutional layer Conv1D_H2, there is a downsampling in one dimension as the scale of the output tensor ts3 decreases from 2 compared to the one ts2 of the previous convolutional layer which is Conv1D_H1 (one goes from 300 to 298), and there is an upsampling in the other dimension as the scale of the output tensor ts3 increases from 2 compared to the one ts2 of the previous convolutional layer which is Conv1D_H1 (one goes from 6 to 12). It is to be noted that the change in dimensions is due to the sliding of the kernels f through the 300 elements. 12 is the number of kernels used for the Convolution.

The batch normalization layer BatchL.2 that follows permits to standardize the first data d1 that goes through this second convolutional layer Conv1D_H2.

The output tensor ts4 is of size 296x10 and serves as an input tensor for the third hidden layer which is the third convolutional layer Conv1D_H3. As, we can see, from the second convolutional layer Conv1D_H2 to the third convolutional layer Conv1D_H3, there is a downsampling in both dimensions of the output tensor ts5 as the scale of the output tensor ts5 decreases compared to the one ts4 of the previous convolutional layer which is Conv1D_H2. The downsampled result is a result of applying kernels convolution.

The batch normalization layer BatchL.3 that follows permits to normalize the first data d1 that goes through this third convolutional layer Conv1D_H3. The output tensor is of size 294x11 and serves as an input tensor for the dropout layer DropL. It is to be noted that batch normalizations are applied after the convolution operations and the Relu activations functions, so it normalizes data going through the next layer.

The three convolutional layers Conv1D_H1, Conv1D_H2, Conv1D_H3 permit to reduce the size of the first input tensor ts1 in the first dimension (one goes from 300 to 294), and to increase the size of the first input tensor ts1 in the second dimension (one goes from 6 to 11).

The dropout layer DropL help to prevent overfitting of the CNN. It serves as a mask that nullifies the contribution of some neurons towards the next layer and leaves unmodified all others neurons. As illustrated in figure 4b, in a non-limitative embodiment, the output tensor ts6 of the dropout layer DropL is of 294x11 and serves as an input tensor for the max pooling layer MaxpL1D that follows.

The max pooling layer MaxpL1D permits to reduce the amount of information while conserving the most prominent information of the previous tensor. It permits to decrease the size of the convolved feature map to reduce computational costs. It also permits to generalize the information extracted by the convolutional filters f. Hence, the max pooling layer MaxpL1D is to reduce the data dimension while keeping the most important information. As illustrated in figure 4b, in a non-limitative embodiment, the output tensor ts7 is of size 147x11 and serves as an input tensor for the flatten layer FlattL. The output tensor is also called pooled feature map.

As illustrated in figure 4b, the flatten layer FlattL takes the output of the previous layer, aka the max pooling layer Maxpm1D, flattens it so as to have a single vector (aka a one-dimensional array) that serves as an input for the following fully connected layer Fc_1 which is the layer Full_L. It means that it reorders the input tensor ts7 in a single column. The output tensor ts8 which is a single vector is of size is 1617 in a non-limitative embodiment.

The Relu activation function that is used within the fully connected layer Full_L permits to set any negative elements to 0.0. The output tensor is of size 15 and serves as an input tensor for the final connected output layer Cont1D_Out.

The following fully connected layer Fc_2 is the final connected output layer Cont1D_Out which has 2 units. It means that the output tensor of the final connected output layer Cont1D_Out is of size 2 as illustrated in figure 4b. It corresponds to two probabilities, a first probability pb1 to be in a first class Cl.0 and a second probability pb2 to be in a second class CI.1. The first class Cl.0 corresponds to a drowsy state, and the second class CI.1 corresponds to a non-drowsy state. The two probabilities pb1, pb2 have a value between 0 and 1.

In order for these two values to add up to 1, in a non-limitative embodiment, the final connected output layer Cont1D_Out uses a Softmax function as an activation function. An activation decides whether a neuron's input to the network is important or not in the process of the prediction. In particular, the Softmax function is used to normalized the output of the CNN to a probability distribution over predicted output classes, here the classes Cl.0 and Cl.1. The values of the two probabilities are therefore complementary. In a non-limitative example, pb1 = 0.4 for class Cl.1 and pb2 = 0.6 for class Cl.0. As we have a binary classification (only two classes), in another non-limitative embodiment, the logistic sigmoid activation can be also used.

It is to be noted that a backpropagation Bp is applied on the whole layers of the CNN so as to propagate an error gradient from the final connected output layer Cont1D_Out to the input convolutional layer Conv1D_In. It permits to the CNN to learn the best values (aka weights) for the filters f to achieve the task in hand, here the classification into the drowsiness state st1 or non-drowsiness state st2. It is to be noted that the backpropagation Bp is applied also on the batch normalization layers BatchL.1, BatchL.2, BatchL.3. For the backpropagation Bp, the cost function used is binary focal cross entropy and the learning rate is of 10e-4 as mentioned before.

Hence, finally, in step E13 illustrated F13(CNN, O1, Cl, st(stl, st2)) in figure 1, the CNN generates first outputs O1 that are a classification of the driver's state st among the drowsiness state st1 and the non-drowsiness state st2.

Hence, there are two classes : Cl.0 = drowsy, and CI.1 = non-drowsy. A first output O1 represents one of the two classes Cl.0, CI.1. One generates one first output O1 that is a classification Cl of the driver's state st. Hence, the CNN generates the probability of the two class Cl.0 and CI.1. It is to be noted that the generation is for each buffer 33 update (new data received from the CAN bus), means each 200ms in the non-limitative given example.

Hence, the CNN permits to take into account a huge amount of first data d1 that a classical driver monitoring system can't.

According to the classifications, a decision can be taken. In the non-limitative example of pb1 = 0.4 for class Cl.1 and of pb2 = 0.6 for class Cl.0, one can deduce that the driver is not drowsy.

Figure 2 illustrates the first computer-implemented method 1 that comprises a further step in a non-limitative embodiment.

In a non-limitative embodiment, the first computer-implemented method 1 further comprises a step E00 illustrated F00(30, d1, MinMax) of normalizing the first data d1 using MinMax normalization. This step is performed before the CNN receives the first data d1. It is applied on the first data d1 of the first input dataset D1. It permits to obtain some first data d1 which values are from 0 to 1. It permits the CNN to learn faster and more efficiently.

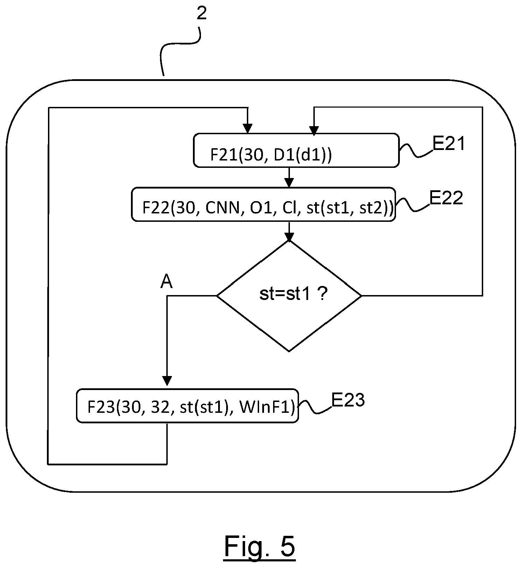

A second computer-implemented method 2 for warning the driver of the vehicle 3 of his drowsiness state is illustrated in figure 5. This second computer-implemented method 2 is performed by the electronic control unit 30 of the vehicle 3.

It comprises the following steps.
In step E21 illustrated F21(30, D1(d1)), the electronic control unit 30 receives as an input for the convolutional neural network CNN the first input dataset D1 comprising first data d1.
In step E22 illustrated F22(30, CNN, O1, Cl, st(stl, st2)), the electronic control unit 30 runs an inference of said CNN. When the inference of said CNN is run, the first computer-implemented method 1 is executed so that said inference CNN generates first outputs O1 that are a classification of the driver's state st among the drowsiness state st1 and the non-drowsiness state st2. While the inference CNN model runs, the steps of the first computer-implemented method 1 are executed.
In step E23 illustrated F23(30, 32, st(st1), wlnF1), if the driver's state st is classified as the drowsy state st1 (branch A illustrated in figure 5), the electronic control unit 30 sends a first warning information wlnF1 to a human machine interface 32 (illustrated in figure 7) of the vehicle 3 to warn the driver of his/her state st and to stop driving and take some rest in a non-limitative example. Then, one goes back to step E21. It is to be noted that as long as the vehicle 3 is moving, the steps E21 to E23 are executed so that the state st of the driver is always controlled.

In a non-limitative embodiments, the first warning information wlnF1 is a displayed message or an audible message or a combination of both. In non-limitative embodiments, the human machine interface 32 is a microphone or a display on the control panel of the vehicle 3.

If the driver's state st is classified as the non-drowsy state st2 (branch B illustrated in figure 5), one goes back to step E21. It is to be noted that even after detecting the drowsy state st1 (branch A illustrated in figure 5), one keeps running the second computer-implemented method 2 and thus, one goes back to step E21)

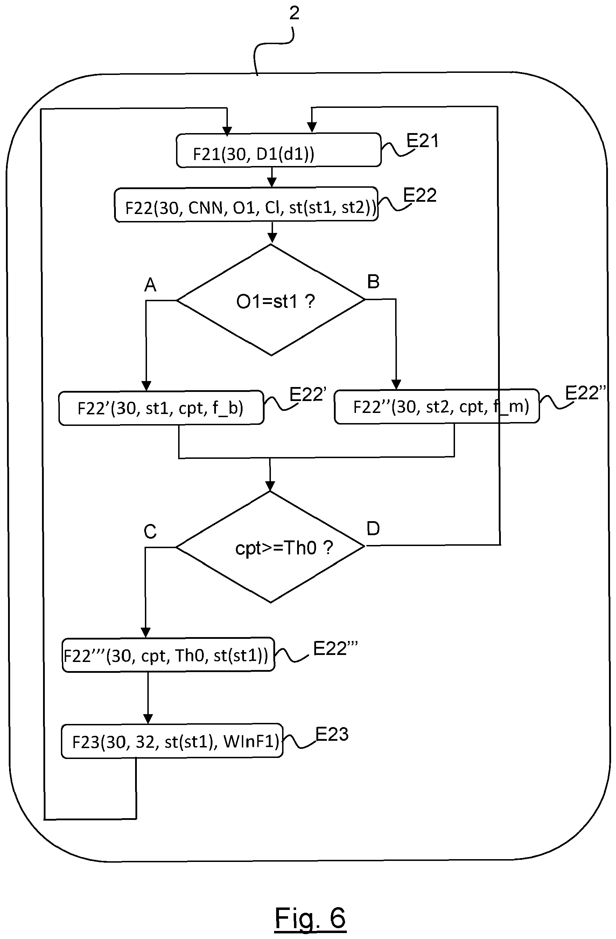

In order to be sure of the state st of the driver, a filtering on the classifications is performed as follows according to a second non-limitative embodiment of the second computer-implemented method 2 illustrated in figure 6.

As illustrated in figure 6, the second computer-implemented method 2 further comprises :
- in step E22' illustrated F22'(30, st1, cpt, f_b), each time the inference CNN generates a classification that corresponds to a drowsiness state st1 (branch A illustrated in figure 6), a filtering counter cpt is increased by the electronic control unit 30 with a filtering bonus f_b. In a non-limitative embodiment, the filtering bonus f_b is equal to 3.
- in step E22" illustrated F22'(30, st1, cpt, f_m), each time the inference CNN generates a classification that corresponds to a non-drowsiness state st2 (branch B illustrated in figure 6),, the filtering counter cpt is decreased by the electronic control unit 30 with a filtering malus f_m. In a non-limitative embodiment, the filtering malus f_m is equal to 1.
- If the filtering counter cpt is above a first filtering threshold Th0 (branch C illustrated in figure 6), classifying by the electronic control unit 30 the driver's state st as the drowsy state st1 (step E22‴ illustrated F22‴(30, cpt, Th0, st(st1))), and thus sending (Step E23) by the electronic control unit 30 the first warning information wlnF1 to a human machine interface 32 of the vehicle 3 to warn the driver of his/her state st and to stop driving and take some rest in a non-limitative example. Then, one goes back to step E21.

If the filtering counter cpt is below the first filtering threshold Th0 (branch D illustrated in figure 6), one goes back to step E21. It is to be noted that as long as the vehicle 3 is moving, the steps E21 to E23 are executed so that the state st of the driver is always controlled.

In a non-limitative embodiment, the first filtering threshold Th0 is superior or equal to 861.

It is to be noted that the second computer-implemented method 2 can be performed by a computer program product Pg. Said computer program product Pg comprises a set of instructions, which when loaded into a computer, causes the computer to carry out the first computer-implemented method 1. Hence, the computer program product Pg is embodied on the non-transitory computer readable storage medium Md having a set of instructions stored therein.

The computer program product Pg is also referred to as a program, software, software application, or code. A computer program product can be deployed in any form, including, but not limited to, as a stand-alone program, or as a module, component, subroutine, or other unit suitable for use in a computing environment.

In non-limitative embodiments, the instructions also called program instructions, may be assembler instructions, instruction-set-architecture (ISA) instructions, machine instructions, microcode, firmware instructions, configuration data for integrated circuitry, or either source code, or object code written in any combination of one or more programming languages, including compiled of interpreted languages, such as procedural programming language or object-oriented programming language.

In non-limitative embodiments, the non-transitory computer readable storage medium Md is an electronic storage device, a magnetic storage device, an optical storage device, an electromagnetic storage device, a semiconductor storage device, or any suitable combination of the foregoing. In non-limitative examples, the computer readable storage medium Md is a read-only memory ROM or the like such as PROM, an erasable programmable read-only memory EPROM or the like such as EEPROM, a Flash memory, a solid-state memory, a DVD, and the like.

In a non-limitative embodiment, the non-transitory computer readable storage medium Md is a memory 31 of the electronic control unit 30 of the vehicle 3 as illustrated in figure 7. Hence, the computer program product Pg (illustrated in figure 7) comprises a set of instructions, which when loaded into said the electronic control unit 30, causes the electronic control unit 30 to carry out the second computer-implemented method 2.

The electronic control unit 30 that runs the inference CNN model is illustrated in figure 7. It comprises a memory 31, and a buffer 33.

The electronic control unit 30 of the vehicle 3 is configured to :
- receive as an input for the convolutional neural network CNN the first input dataset D1 comprising first data d1, said convolutional neural network CNN comprising a set of hyperparameters Hp (function illustrated f301(30, D1(d1), CNN)),
- run an inference model of said convolutional neural network CNN (also called inference CNN model) so that said convolutional neural network CNN generates first outputs O1 that are a classification of the driver's state st among the drowsiness state st1 and the non-drowsiness state st2 (function illustrated f302(30, CNN, O1, st(stl, st2))),
- if the driver's state st is classified as the drowsy state st1, send a first warning information wlnF1 to a human machine interface 32 of the vehicle 3 (function illustrated f303(30, 32, st(st1), wlnF1)).

In a non-limitative embodiment, the electronic control unit 30 is further configured to :
- normalize the first data d1 using MinMax normalization (function illustrated f304(30, d1, MinMax)).

The electronic control unit 30 is further configured to :
- increase a filtering counter cpt with a filtering bonus f_b each time the inference convolutional neural network CNN generates a classification that corresponds to a drowsiness state st1, (function illustrated f305(30, st1, cpt, f_b)),
- decrease the filtering counter cpt with a filtering malus f_m each time the inference convolutional neural network CNN generates a classification that corresponds to a non-drowsiness state st2 (function illustrated f306(30, st2, cpt, f_m)),
- if the filtering counter cpt is above a first filtering threshold Th0, classifying the driver's state st as the drowsy state st1 (function illustrated f307(30, cpt, Th0, st(st1))), and thus send a first warning information wlnF1 to a human machine interface 32 of the vehicle to warn the driver of his/her state st and to stop driving and take some rest in a non-limitative example (function illustrated f303(30, 32, st(st1), wlnF1)).

It is to be understood that the present invention is not limited to the aforementioned embodiments and variations and modifications may be made without departing from the scope of the invention. In this respect, the following remarks are made. All statements herein reciting principles, aspects, and embodiments of the invention, as well as specific examples thereof, are intended to encompass equivalents thereof.

Hence, in a non-limitative embodiment, before running the inference CNN model, there is a deactivation of the CNN during a first time duration when the vehicle yaw rate is above a yaw rate threshold. Indeed, in this case, one is sure that the driver is not drowsy, so it is not necessary to run the inference CNN. In a non-limitative embodiment, the first time duration is 90 seconds.

Hence, in a non-limitative embodiment, before running the inference CNN, there is a deactivation of the CNN during a second time duration when the vehicle blinker signal is activated. Indeed, in this case, one is sure that the driver is not drowsy, so it is not necessary to run the inference CNN. In a non-limitative embodiment, the first time duration is 60 seconds.

Hence, in a not limitative embodiment, a filter f also comprises a bias.

Hence, some embodiments of the invention may comprise one or a plurality of the following advantages:
- it permits to obtain a detection of the state st of a driver with good performances,
- thanks to the use of the CNN model for the detection of the state st of a driver, one doesn't need additional equipment in the vehicle like a driver monitoring system camera for monitoring the driver's face, or a sensor to capture biological signals to provide security to the driver. One only needs the vehicle signals and a frontal exterior camera (for the lanes' signals),
- it permits to reduce the cost of the detection, as one reduces the number of equipment needed,
- it is a solution that is based on real world dataset and not on simulator or synthetic data. This means the performances obtained are based on a real scenario with real driving environment, which is more robust than systems based on synthetic or artificial data,
- it is a solution that solves the problem of classical driver monitoring systems that only meets specific conditions for a specific dataset. With the use of a deep learning model such as the CNN model, the first data d1 are used as input so that the CNN model can learn on these first data d1. Moreover, the CNN model is not limited to a given vehicle and thus the first computer-implement method 1 can be embedded inside different types of vehicles, and the same goes for the second computer-implement method 2,
- by using a 1D-CNN architecture, there is no need to calculate features that are supposed to capture drowsiness behavior patterns (such as steering wheel micro corrections and speed variability for example). However, we just inject the raw signals (aka the first data d1) and the CNN model layers will learn by their own the patterns that differentiate between drowsiness st1 and non-drowsiness st2 states.

## Claims

1. A first computer-implemented method (1) to detect a state (st) of a driver of a vehicle among a drowsiness state (st1) and a non-drowsiness state (st2), said first computer-implemented method (1) comprising :
- receiving as an input by a convolutional neural network (CNN) a first input dataset (D1) comprising first data (d1), said convolutional neural network (CNN) comprising a set of hyperparameters (Hp),
- processing said first data (d1) through a plurality of layers (L) of said convolutional neural network (CNN),
- generating by said convolutional neural network (CNN) first outputs (O1) that are a classification of the driver's state (st) among the drowsiness state (st1) and the non-drowsiness state (st2).

2. A first computer-implemented method (1) according to claim 1, wherein said first data (d1) are :
- a vehicle speed (d1.1), and/or
- a vehicle steering wheel angle (d1.2), and/or
- a vehicle brake pedal pressure (d1.3), and/or
- a vehicle acceleration pedal pressure (d1.4), and/or
- a vehicle yaw rate (d1.5), and/or
- a vehicle position on a road lane (d1.6).

3. A first computer-implemented method (1) according to any of the preceding claims, wherein said first input dataset (D1) comprises about a number (Ns) of sequences of 300 by 6 elements, each sequence being acquired during a history of one 1 minute.

4. A first computer-implemented method (1) according to the preceding claim, wherein the first input dataset (D1) is updated each N milliseconds using a sliding window (w), with N integer.

5. A first computer-implemented method (1) according to the preceding claim, wherein N is subsequently equal to 200 milliseconds.

6. A first computer-implemented method (1) according to any of the preceding claims, wherein said hyperparameters (Hp) are :
- a loss function (Hp.1) that is a binary focal cross entropy,
- an optimizer (Hp.2) that is an Adam optimizer,
- a learning rate (Hp.3) of 10e-4
- an epoch number (Hp.4) of 23.

7. A first computer-implemented method (1) according to any of the preceding claims, wherein said first computer-implemented method (1) further comprises normalizing the first data (d1) using MinMax normalization.

8. A first computer-implemented method (1) according to any of the preceding claims, wherein said convolutional neural network (CNN) comprises:
- one input convolutional layer (Conv1D_In),
- three convolutional layers (Conv1D_H1, Conv1D_H2, Conv1D_H3),
- one fully connected layer (Full_L),
- one final connected output layer (Cont1D_Out).

9. A first computer-implemented method (1) according to the preceding claim, wherein :
- said three convolutional layers (Conv1D_H1, Conv1D_H2, Conv1D_H3) use each a Relu activation function and by a batch normalization layer (BatchL.1, BatchL.2, BatchL.3), each three convolutional layers comprising respectively 12, 10 and 11 filters (f) of size 3,
- said fully connected layer Full_L of 15 units with a Relu activation function, said final connected output layer (Cont1D_Out) has 2 units and is using a Softmax function.

10. A first computer-implemented method (1) according to the preceding claims 8 or 9, wherein said convolutional neural network (CNN) further comprises :
- one dropout layer (DropL), and
- one max pooling layer (MaxL).

11. A first computer-implemented method (1) according to any of the preceding claims, wherein said convolutional neural network (CNN) further comprises an additional fully connected layer that is a flatten layer (FlattL) that is applied before the fully connected layer (Full_L).

12. A first computer-implemented method (1) according to any of the preceding claims, wherein a backpropagation (Bp) that is applied on the whole layers so as to propagate an error gradient from the final connected output layer (Cont1D_Out) to the input convolutional layer (Conv1D_In).

13. A second computer-implemented method (2) for warning a driver of a vehicle (3) of his drowsiness state (st1), said second computer-implemented method (2) comprising :
- receiving as an input for a convolutional neural network (CNN) a first input dataset (D1) comprising first data (d1), said convolutional neural network (CNN) comprising a set of hyperparameters (Hp),
- running an inference of said convolutional neural network (CNN) so as to execute said first computer-implemented method (1) according to any of the preceding claims 1 to 12 so that said inference convolutional neural network (CNN) generates first outputs (O1) that are a classification of the driver's state (st) among the drowsiness state (st1) and the non-drowsiness state (st2),
- if the driver's state (st) is classified as the drowsy state (st1), sending a first warning information (wlnF1) to a human machine interface (32) of the vehicle (3).

14. A second computer-implemented method (2) according to the preceding claim, wherein said second computer-implemented method (2) further comprising :
- each time the inference convolutional neural network (CNN) generates a classification that corresponds to a drowsiness state (st1), a filtering counter (cpt) is increased by the electronic control unit (30) with a filtering bonus (f_b),
- each time the inference convolutional neural network (CNN) generates a classification that corresponds to a non-drowsiness state (st2), the filtering counter (cpt) is decreased by the electronic control unit (30) with a filtering malus (f_m),
- if the filtering counter (cpt) is above a first filtering threshold (Th0), classifying the driver's state (st) as the drowsy state (st1).

15. Electronic control unit (4) for a vehicle (3), wherein said electronic control unit (4) being configured to :
- receive as an input for a convolutional neural network (CNN) a first input dataset (D1) comprising first data (d1), said convolutional neural network (CNN) comprising a set of hyperparameters (Hp),
- run an inference of said convolutional neural network (CNN) so that said convolutional neural network (CNN) generates first outputs (O1) that are a classification of the driver's state (st) among the drowsiness state (st1) and the non-drowsiness state (st2),
- if the driver's state (st) is classified as the drowsiness state (st1), send a first warning information (wlnF1) to a human machine interface (32) of the vehicle (3).
